# EUROPEAN PATENT APPLICATION

(11) **EP 1 723 905 A2**
(43) Date of publication of application: **22.11.2006**
(21) Application number: 06113756.8
(22) Date of filing: 10.05.2006
(51) Int. Cl.: A61B 5/0215, A61M 1/36

(54) **Device for non-invasive measurement of the pressure of a fluid, particularly for measuring blood pressure in medical equipment**

(30) Priority: 20.05.2005 IT MO20050125
(71) Applicant: M.D. MICRO DETECTORS S.p.A., I-41100 Modena (IT)
(72) Inventor: Del Monte, Mauro, 41100, MODENA (IT)
(74) Representative: Modiano, Micaela Nadia

(57) **Abstract**

A device (1) for non-invasive measurement of the pressure of a fluid, particularly for measuring blood pressure in medical equipment, comprises a first tubular element (2), which is at least partially flexible and is adapted for the passage of an internal fluid (3), and a chamber (5) for containing an interface fluid (6), which is at least partially in contact with the first tubular element (2), the containment chamber (5) being connectable to a pressure sensor in order to define a value of the pressure of the internal fluid by means of a measurement of the pressure of the interface fluid (6).

## Description

The present invention relates to a device for non-invasive measurement of the pressure of a fluid, particularly for measuring blood pressure in medical equipment.

Medical equipment moves blood by means of pumps, which allow to extract the blood from the patient; this is followed by the execution of a specific process on the blood, which is subsequently reinjected into the patient.

This method is used in many kinds of therapeutic treatment and of treatment of other kinds, such as for example in equipment for hemodialysis, suitable to eliminate certain toxins from blood, or in the case of a plasmapheresis process, aimed at separating plasma from blood.

During the steps for extraction, transfer and reinjection, it is crucially important to measure continuously the blood pressure in certain points of the machine.

Preventive measures intended to reduce the possibility of damage to the patient provide, for example, for monitoring of the pressure at the withdrawal point and at the injection point. Additional measurement points can further be defined according to the type of process to be performed on the blood.

Devices for measuring the pressure inside medical equipment are known.

In particular, a first known device has a small reservoir, inside which the blood is made to flow, said blood being introduced by means of a tube which is connected to an inlet port and being extracted through an outlet port.

Air is present inside the reservoir, and in static conditions the pressure of such air is equivalent to the pressure of the blood. In this manner, it is possible to measure the pressure of the blood by measuring the pressure in the vicinity of an air outlet port to which a pressure sensor is connected.

This device is not free from drawbacks, the first of which is the occurrence of direct contact between air and blood, with the consequent need to provide safety measures in order to protect the patient. These measures must provide for the use of air filters upstream of the air intake pump as well as suitable checks to prevent the formation of the air bubbles within the blood or in any case prevent the injection of any generated bubbles into the patient.

In addition to the above, there is the drawback due to the process of coagulation of blood in contact with air.

Further, pressure measurement occurs by means of a compressible medium, leading to a measurement which is correct by definition only in static conditions and forcing the use of suitable compensations in the case of measurements made in dynamic conditions.

Moreover, the location of the pressure sensor proximate to the air outlet port, or in any case along a tube which is connected to said outlet port, does not ensure the absence of contaminations of the sensor on the part of the blood and vice versa.

Another drawback is the need to draw more blood from the patient than strictly necessary, said blood being collected within the reservoir in order to perform the measurement.

Some of the problems described above are solved by interposing a flexible membrane between the chamber that contains the blood and the air outlet port. In this case, the possibility of contaminations between the blood and the pressure sensor is in fact limited.

Currently, the interface fluid used by these devices is still air; therefore, pressure transmission occurs with limited effectiveness, and the execution of the device further requires relatively high costs.

In order to achieve good elasticity of the membrane, said membrane is made of extremely thin materials, which can deteriorate easily in a short time.

A second known type of device for measuring the pressure of a fluid uses a tube which comprises a portion which is rendered suitably flexible by means of a particular shape. A first embodiment uses a wider shape of the flexible portion, while a second embodiment provides a concertina shape.

Variations in the pressure of the blood inside the tube lead respectively to variations in the diameter and length of the flexible portion.

Although this device solves most of the problems listed above, it constrains the pressure measurement to the elasticity of the material, yielding a scarcely satisfactory performance.

A further alternative to the above devices uses a buffer medium, which is a gel inserted during manufacture.

The gel (usually a silicone material) covers the sensing element of the pressure sensor and acts as an interface.

Although it provides a good performance, in this case it is difficult to ensure the lack of risk of blood contamination.

Further, this solution forces destruction of the measurement device at the end of the treatment.

The aim of the present invention is to eliminate the above mentioned drawbacks of the background art, by providing a device which allows correct and non-invasive measurement of the pressure of a fluid.

Within this aim, an object of the present invention is to allow a blood pressure measurement within medical equipment which ensures a high level of safety for the patient.

Another object of the invention is to avoid dangers of contamination among different patients, allowing the replacement of components which can be contaminated, allowing to keep the measurement sensors reusable without any risk, and further ensuring low-cost production of these components.

Another object of the invention is to allow to withdraw from the body of the patient only the amount of blood that is strictly necessary for the functions of the machine, without requiring additional quantities to measure the pressure.

Another object of the invention is to allow to provide a system without any contact with air, so as to reduce or even eliminate the risk of the formation of bubbles, foam and clots.

Another object of the present invention is to provide a measurement system which is capable of self-monitoring, checking its own measurement sensitivity and detecting faults.

Within this aim, another object of the present invention is to provide a device which is simple, relatively easy to provide in practice, safe in use, effective in operation, and has a relatively low cost.

This aim and these and other objects which will become better apparent hereinafter are achieved by the present device for non-invasive measurement of the pressure of a fluid, particularly for measuring blood pressure in medical equipment, characterized in that it comprises a first tubular element, which is at least partially flexible and is suitable for the passage of an internal fluid, and a chamber for containing an interface fluid, which is at least partially in contact with said first tubular element, said containment chamber being connectable to a pressure sensor in order to define a value of the pressure of said internal fluid by means of a measurement of the pressure of said interface fluid.

Further characteristics and advantages of the present invention will become better apparent from the following detailed description of a preferred but not exclusive embodiment of a device for non-invasive measurement of the pressure of a fluid, illustrated by way of non-limiting example in the accompanying drawings, wherein:
Figure 1 is a front sectional view of the device according to the invention;
Figure 2 is a side sectional view of the device according to the invention;
Figure 3 is a view, according to the invention, of the forces which act within the device during the flow of the fluid whose pressure is measured;
Figure 4 is a side elevation view of the device according to the invention.

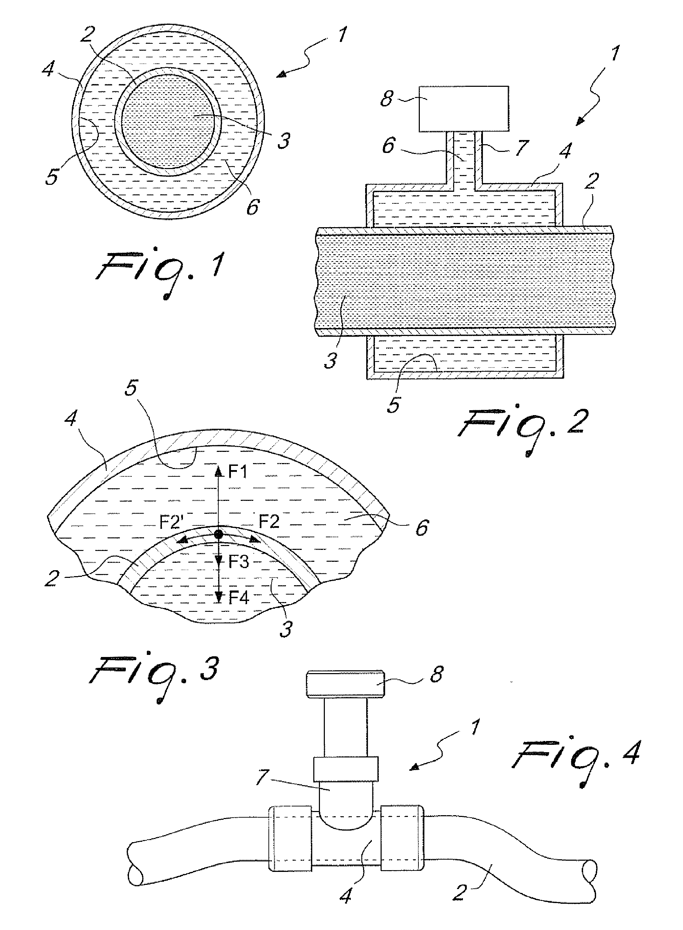

With reference to the figures, the reference numeral 1 generally designates a device for non-invasive measurement of the pressure of a fluid.

In particular, the device 1 is applied in the field of medical equipment, specifically for measuring the pressure of a medical fluid.

The device 1 comprises a first tubular element 2, which is at least partially flexible, for the flow of an internal fluid 3. When the internal fluid 3 flows, the hydrostatic pressure applied to the internal surfaces tends to cause the expansion of the walls of the tube along the flexible portion.

Further, the device being considered provides for the presence of a second tubular element 4, which contains at least partially the flexible portion of the first tubular element 2.

In particular, the first tubular element 2 is made entirely of flexible material, and a portion thereof is inserted within the second tubular element 4, which is made of rigid material.

A containment chamber 5 for an interface fluid 6 is formed between the tubular elements 2 and 4 and comprises a connecting inlet port 7, which is located on the wall of the second tubular element 4 and to which a pressure sensor 8 can be connected.

If the characteristics of the materials that compose the two tubes and the characteristics of the interface fluid 6 within the interspace 5 are known, it is possible to determine the pressure of the internal fluid 3 by means of a measurement, on the part of the sensor 8, of the pressure of the interface fluid 6.

As shown in Figure 4, which illustrates the forces that act on a particular point which belongs to the flexible wall of the first tubular element 2, the pressure applied following the flow of the fluid 3 being measured generates a force F1, which is perpendicular to the surface and tends to dilate the walls of the first tubular element 2. The forces designated by F2 and F2' are reaction forces which are a consequence of the traction of the wall of the tube.

The forces F3 and F4, respectively the reaction force caused by the compression of the material and the force applied by the external interface fluid 6, are shown in a direction which is perpendicular to the wall of the internal tube and with an opposite orientation with respect to the force F1.

Preferably, the interface fluid 6 is an incompressible fluid, such as to allow a lower expansion of the first tubular element 2 and ensure, in conditions of equilibrium, a pressure which is substantially equal to the pressure of the internal fluid 3.

In particular, the first tubular element 2 is made of a flexible material having a high compressibility modulus and Young modulus respectively at the sensitivity required by the pressure sensor 8.

This, together with the presence of an incompressible fluid within the interspace 5, ensures high sensitivity of the measurement made by the pressure sensor 8.

With particular reference to applications of the medical type, the incompressible fluid is preferably a physiological solution.

As an alternative, if the interface fluid 6 is a compressible fluid, the expansion of the first tubular element 2 is contrasted predominantly by the elastic forces of the walls of the tube, with a consequent variation of the pressure of the interface fluid 6 which is less than the pressure variations of the internal fluid 3.

With reference to the structure of the tubular elements, the first tubular element 2 and/or the second tubular element 4 have a substantially cylindrical shape.

In particular, both the first tubular element 2 and the second tubular element 4 are substantially cylindrical and the first tubular element 2 is contained within the second tubular element 4 with a substantially coaxial arrangement.

Conveniently, the tubular elements 2 and 4 can be produced as an integrated unit during their manufacture, or the tubular element 4 can be constituted by an independent structure which can be applied at the time of use and is available for subsequent reuses.

In particular, in the embodiment of the invention being considered, the second tubular element 4 is constituted by a three-way union, in the specific case by a T-shaped union for compressed air circuits, within which the first tubular element 2, constituted by a flexible tube for moving the blood, is inserted.

The interspace 5 between the two tubes is filled with water and the device 1 is connected by means of a third inlet port 7 of the three-way union to a pressure sensor 8, which in the specific case is a pressure gage, which need not be an integral part of the device 1 but might be connected thereto at the time of use by means of a pneumatic coupling.

This configuration of the device 1 allows a measurement of the pressure on the part of the sensor on the order of 100 Pa, ensuring an accurate pressure measurement according to the parameters required in the field of medical equipment.

If the interface fluid 6 is constituted by water or, more generally, by an incompressible fluid, interspace filling means are provided. Filling with the incompressible fluid occurs completely and so as to avoid the presence of air bubbles inside the interspace 5.

The filling means comprise a container for the water to be introduced, in which it is possible to generate a partial vacuum, and a system of valves and cocks, which are connected to the rigid tube, suitable to generate vacuum inside the interspace 5 and completely fill said interspace with the water inside the container.

As an alternative, the interspace 5 between the two tubes can be filled with air. In this case, a lower resolution of the pressure measurement made by the sensor is obtained. To reach the particular level of precision of the measurement that is required for measuring blood pressure in medical equipment, it is possible to introduce a more sensitive pressure sensor and/or a more accurate electronic system for conditioning the signal and/or means and a verification procedure capable of detecting the characteristics of the system.

In the specific case of the application of the device 1 in the field of medical equipment, one possible implementation has, in addition to the pressure sensor, a stabilization system which is suitable to set a constant and known pressure of the interface fluid 6, constituted for example by an appropriately provided actuator capable of inducing a volume variation or, more generically, a variation of a correction value.

The volume variation, or the correction value, is the parameter which is proportional to the pressure to be measured and can be checked by means of a control system.

Systems of this kind allow to subject the device 1 periodically to forces and check its integrity and precision.

The operation of the present invention is as follows.

The device 1 can be applied along a blood transfer circuit of a piece of medical equipment. The first tubular element 2, constituted by a flexible tube suitable for such movement, is inserted within the second tubular element 4.

The interspace 6 is filled completely with water by means of a vacuum procedure which allows effective air extraction.

When the medical apparatus is working, and therefore when the blood of a patient flows through the movement circuit, any variations of the blood pressure lead to variations of the forces applied to the walls of the flexible tube. This leads to an expansion of the tube, with a consequent variation of the pressure of the water within the interspace 5.

The variation of the pressure of the water is detected by a pressure gauge connected to the second tubular element 4 by means of the port 7.

In practice it has been found that the described invention achieves the intended aim and objects, and in particular the fact is stressed that the device allows correct and non-invasive measurement of the pressure of a fluid.

The invention thus conceived is susceptible of numerous modifications and variations, all of which are within the scope of the appended claims.

In this regard, it should be noted that the tubular structure, which has a coaxial geometry, is not the only one that allows correct operation but is only the one that has maximum dimensional efficiency.

The efficiency of pressure transmission depends in fact mainly on the area of the membrane, and it is therefore possible to provide the device by providing a containment chamber which is not completely wrapped around the first tubular element but is instead placed in contact with a portion thereof, allowing the device to be applied by simply arranging the parts in contact.

All the details may further be replaced with other technically equivalent elements.

In practice, the materials used, as well as the contingent shapes and dimensions, may be any according to requirements without thereby abandoning the scope of the protection of the appended claims.

The disclosures in Italian Patent Application No. M02005A000125 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device for non-invasive measurement of the pressure of a fluid, particularly for measuring blood pressure in medical equipment, **characterized in that** it comprises a first tubular element, which is at least partially flexible and is adapted for the passage of an internal fluid, and a chamber for containing an interface fluid, which is at least partially in contact with said first tubular element, said containment chamber being connectable to a pressure sensor in order to define a value of the pressure of said internal fluid by means of a measurement of the pressure of said interface fluid.

2. The device according to claim 1, **characterized in that** it comprises a second tubular element, which contains at least partially said first tubular element, said containment chamber being formed between said tubular elements.

3. The device according to one or more of the preceding claims, **characterized in that** said second tubular element is made of rigid material.

4. The device according to one or more of the preceding claims, **characterized in that** said first tubular element and/or said second tubular element have a substantially cylindrical shape.

5. The device according to one or more of the preceding claims, **characterized in that** both said first tubular element and said second tubular element have a substantially cylindrical shape, said first tubular element being contained within said second tubular element with a substantially coaxial arrangement.

6. The device according to one or more of the preceding claims, **characterized in that** said second tubular element comprises an inlet port for the connection of said pressure sensor.

7. The device according to one or more of the preceding claims, **characterized in that** said second tubular element comprises a three-way union for compressed air.

8. The device according to one or more of the preceding claims, **characterized in that** said first tubular element is entirely made of flexible material.

9. The device according to one or more of the preceding claims, **characterized in that** said interface fluid is an incompressible fluid.

10. The device according to one or more of the preceding claims, **characterized in that** said incompressible fluid is a physiological solution.

11. The device according to one or more of the preceding claims, **characterized in that** said incompressible fluid is water.

12. The device according to one or more of the preceding claims, **characterized in that** said interface fluid is a compressible fluid.

13. The device according to one or more of the preceding claims, **characterized in that** said pressure sensor is a pressure gauge.

14. The device according to one or more of the preceding claims, **characterized in that** it comprises a stabilization system, which is adapted to force a constant known pressure of said interface fluid, said system being associated with a control system which is suitable to check variations in the measurement of the pressure of said interface fluid which can be detected by said pressure sensor.

15. The device according to one or more of the preceding claims, **characterized in that** it comprises filling means which are adapted to fill completely and without air residues said interface with said interface fluid.
